(19) **Europäisches Patentamt**

**European Patent Office**

**Office européen des brevets**

(11) Veröffentlichungsnummer: **0 204 786 B1**

(12) **EUROPÄISCHE PATENTSCHRIFT**

(45) Veröffentlichungstag der Patentschrift: **02.12.92**

(51) Int. Cl.5: **A61F 2/30**, A61L 27/00

(21) Anmeldenummer: **86900132.1**

(22) Anmeldetag: **16.12.85**

(86) Internationale Anmeldenummer:
**PCT/EP85/00711**

(87) Internationale Veröffentlichungsnummer:
**WO 86/03671 (03.07.86 86/14)**

Teilanmeldung 92108948.8 eingereicht am
16/12/85.

---

(54) **KNOCHENERSATZWERKSTOFF UND SEINE VERWENDUNG.**

---

(30) Priorität: **14.12.84 DE 3445711**

(43) Veröffentlichungstag der Anmeldung:
**17.12.86 Patentblatt 86/51**

(45) Bekanntmachung des Hinweises auf die
Patenterteilung:
**02.12.92 Patentblatt 92/49**

(84) Benannte Vertragsstaaten:
**AT BE CH DE FR GB IT LI LU NL SE**

(56) Entgegenhaltungen:
**EP-A- 0 011 809      EP-A- 0 016 906
EP-A- 0 018 496      WO-A-83/03967
CH-A- 611 794        CH-A- 643 732
DE-A- 2 022 498      DE-A- 2 620 890
DE-A- 2 742 128      DE-A- 2 917 037
DE-A-25 028 84       DE-B- 2 205 808
US-A- 3 855 638**

**Chemical Abstracts, Band 95, Nr. 14, 5. Oktober 1981, Columbus, Ohio (US) see page**

372, abstract 121173d, & SU, A, 833238
(VARAVA et al.) 30. Oktober1981

(73) Patentinhaber: **DRAENERT, Klaus, Dr.med.
Gabriel-Max-Strasse 3
W-8000 München 90(DE)**

(72) Erfinder: **DRAENERT, Klaus, Dr.med.
Gabriel-Max-Strasse 3
W-8000 München 90(DE)**

(74) Vertreter: **Vossius & Partner
Siebertstrasse 4 P.O. Box 86 07 67
W-8000 München 86(DE)**

---

## Beschreibung

Die Erfindung betrifft einen Knochenersatzwerkstoff und seine Verwendung.

Es wurde seit jeher versucht, Knochendefekte nach Knochenverletzungen oder chirurgischen Ausräumungen wieder aufzufüllen, da es eine Erfahrungstatsache darstellte, daß Knochen zu seiner Regeneration lange Zeit benötigt und daß erst nach vollständiger Heilung des Knochens die Funktion einer Extremität wiederhergestellt ist. Große Knochendefekte können jedoch kaum noch so verheilen, daß die ursprüngliche Funktion wiedererlangt werden kann, weswegen seit alters her versucht wurde, Knochenersatzwerkstoffe, sei es durch Transplantate oder durch Implantate, zu verwenden.

Leider war die Suche nach den Materialien, die sich hierfür eignen, noch wenig effektiv. Bei Verwendung autologen Materials, d.h. von Knochentransplantaten desselben Patienten, ist man in der Masse beschränkt, da als Knochenspender nur bestimmte Bereiche des Bewegungsapparates zur Verfügung stehen. Homologes Material hatte bislang die Schranken der Immunreaktion, welche bis zum heutigen Tage nicht bewältigt werden konnten. Aufbereitetes homologes und heterologes Knochenmaterial kann zwar in beschränktem Maße für den Knochenersatz verwandt werden, wird aber, wie experimentell nachgewiesen werden konnte, vom Körper nicht voll akzeptiert und auch nicht vollständig eingebaut.

Man war daher bestrebt, durch weitere Behandlung von tierischen Knochen ein Material zu entwickeln, das in der Lage war, als Knochenersatzwerkstoff Defekthöhlen auszufüllen und diese Defekte von der Masse aus zu überbrücken. Bei all diesen Versuchen ging man davon aus, daß möglichst die Struktur der Knochenwabe erhalten werden sollte.

Alle Verfahren und alle im Handel befindlichen Präparate, mit Ausnahme der heterologen Knochenspäne, den sog. "Cialitspänen" oder auch "Kieler Knochenspänen", bereiten tierischen Knochen oder auch andere Kollagene von Tieren in der Weise auf, daß sie entmineralisiert und durch verschiedene chemische Verfahren ihrer Antigenität beraubt werden. Die so erhaltenen Kollagene werden zwar vom Körper eingebaut und sind auch resorbierbar, eignen sich jedoch weder für die Aufnahme von biomechanischen Kräften, noch dienen sie als stabiles Stützgerüst.

Aus diesem Grunde wurde immer wieder versucht, Werkstoffe zu schaffen, die die Eigenschaften der Knochensubstanz haben, nämlich Stützfunktion zu übernehmen und resorbierbar zu sein. In erster Linie wurden hierfür verschiedene gesinterte Tricalciumphosphate oder Apatite verwandt.

Es wurde auch versucht, durch Strukturierung einer Implantatoberfläche das Einheilen von z.B. Gelenkendoprothesen zu erleichtern. Das Einheilen dieser Prothesen ist vergleichbar mit dem Knocheneinwuchs in Knochenersatzimplantatwerkstoffe, weswegen an die Beschichtung einer Prothese, ihre Struktur und Oberflächenbeschaffenheit, ähnliche Anforderungen gestellt werden. Das Problem des Knocheneinwuchses und der Knochendefektheilung konnte jedoch bislang nicht befriedigend gelöst werden. Werkstoffe zum Knochenersatz auf der Basis strukturierter Kollagene, wie in der DE-A-28 54 490 vorgeschlagen, zeigen keine ausreichend knochenbildende Wirkung.

Zum einen geben sie die Struktur vor, die der Knochen einnehmen sollte, nämlich Bälkchenstruktur, was lediglich durch äußere und innere Anlagerung verstärkte und verdickte Knochenbälkchen ergibt, aber keine normale Knochenarchitektur zuläßt. Zum anderen haben diese Werkstoffe aufgrund der Demineralisierung keine mechanische Festigkeit mehr, was die fehlende Knocheninduktion erklärt und eine Stützfunktion ausschließt. Gesinterte Werkstoffe dagegen sind kaum innerhalb nützlicher Zeit resorbierbar.

Die Probleme beim Gelenkersatz entsprechen denen des Knochenersatzes mit der Ergänzung durch die Tatsache, daß die Grenzzone einer Prothese noch wesentlich stärker beansprucht wird als das Interface zu einem Knochenersatzwerkstoff. Prothesen, d.h. künstliche Gelenke, werden in der Regel mit einem Stift oder Schaft (Verankerungsteil) im Knochen verankert oder diesem aufgesetzt (vgl. Journal of Bone and Joint Surgery, Bd.21 (1939), Seite 269 - 288).

Sehr weit verbreitet ist die Methode, z.B. ein Hüftgelenk dadurch zu ersetzen, daß ein metallischer Vollschaft in die Knochenmarkshöhle versenkt und dort mit einem Zweikomponenten-Kunststoff ("Knochenzement") verankert wird (J. Bone Joint Surg., Bd.42 B (1960), Seite 28 - 30). Die Verträglichkeit und biomechanische Festigkeit der bekannten Knochenzemente ist jedoch unbefriedigend.

In der Regel wird es bei diesen Vorgängen notwendig, die Oberfläche des Prothesenschaftes zu vergrößern, z.B durch wellen- oder sägezahnartige Oberflächengestaltungen; vgl. DE-C-837 294. Aus der DE-A-2 127 843 ist eine poröse Metallbeschichtung bekannt, die mit dem Basiskörper desselben Metalles fest verbunden ist, und die die Oberfläche vergrößern und den Knocheneinwuchs ermöglichen soll . Bei den bekannten Beschichtungen erfolgt ein Einwuchs des Knochens nur unter bestimmten Bedingungen. Es gelingt nicht, reproduzierbare, auf alle Patienten übertragbare Ergebnisse mit derartigen Prothesenoberflächen zu erreichen. Für die Sicherung des knöchernen Einwuchses und die prognostisch erfaßbare Verankerung sind somit noch andere Faktoren verantwortlich.

Erfindungsgemäß wurde festgestellt, daß es im wesentlichen zwei Faktoren gibt, die zum einen den gewünschten Knocheneinwuchs induzieren und zum anderen die Morphologie einer tragenden oder nicht tragenden Knochenstruktur und die damit zusammenhängende Möglichkeit, auftretende Belastungen störungsfrei aufzunehmen, bestimmen können. Es ist dies einerseits die Morphologie der Oberflächenstruktur im Verankerungsteil und andererseits die chemische Zusammensetzung (Chemismus) seiner Oberfläche.

Bei Prüfung der Frage, inwieweit die einzelne knochenbildende Zelle, der Osteoblast, bzw. der Zellverband, das Osteoblastenlayer, durch verschiedene morphologische oder chemisch sich unterscheidende Strukturen oder Stoffe zur Knochenbildung bzw. zum Aufbau bestimmter Knochenbälkchen induziert werden können, hat es sich gezeigt, daß bezüglich der knochenbildenden Zelle eine bestimmte morphologische Struktur im Verankerungsteil die Knochenbildung stark induziert, während es im Hinblick auf den Zellverband eine andere bestimmte morphologische Struktur ist, die zur verstärkten Bildung eines tragenden Knochenbälkchens führt. Andererseits muß man die im Hinblick auf die zur Erzielung optimaler statischer Ergebnisse notwendigen bestimmten Ausgestaltungen (Topographie) der Implantatverankerung bei der Gesamtkonstruktion berücksichtigen, wobei man die gesamte Beanspruchung und den Bewegungsablauf eines Gelenkes im Hinblick auf ein Prothesendesign, das für die Krafteinleitung entscheidend ist, ebenfalls zu berücksichtigen hat.

Ausgehend von den vorstehenden, der Erfindung zugrundeliegenden Befunden und Erkenntnissen über die Knocheninduktion und Morphologie als Kriterien der Formgebung lassen sich vier Dimensionen für die Strukturierung des Knochenersatzwerkstoffes definieren, die nachstehend als Struktur I. - IV.Ordnung bezeichnet werden. Die Struktur I.Ordnung entspricht nach dieser Definition dem äußeren Implantatdesign, z.B. auch der Ausgestaltung des Verankerungsteils einer Prothese. Die Struktur II.Ordnung stellt bereits eine Gestaltung der Oberfläche (Topographie) dar. Beispielsweise werden mit Struktur II.Ordnung besondere Oberflächengestaltungen, wie eine wellenartige Oberflächengestaltung oder sägezahnartige oder treppenartige Gestaltung des Prothesenschaftes bezeichnet. Diese Oberflächenstrukturierungen gemäß einer Struktur II.Ordnung sollen den Vorgang der mechanischen Verankerung unterstützen und die Verankerungsoberfläche im Hinblick auf ihre Beanspruchung differenzieren. Die Struktur III.Ordnung stellt nach der vorliegenden Definition bereits die Mikrostruktur der Oberflächen dar. Sie umfaßt beispielsweise Oberflächenausgestaltungen, z.B. kleine Kugeln bis in den mm-Bereich.

Schließlich wird als Struktur IV.Ordnung die Ultrastruktur mit einer Größenordnung von etwa 20 $\mu$m bezeichnet.

Aus der DE-A-27 30 004 ist ein Verankerungsteil für Knochenendoprothesen, insbesondere Prothesenzapfen bekannt, dessen Oberfläche eine Vielzahl von einstückig mit einem Basiskörper verbundenen, voneinander durch Zwischenräume getrennten Vorsprüngen aufweist, der dadurch gekennzeichnet ist, daß der zwei benachbarte Vorsprünge trennende Zwischenraum mindestens eine Engstelle aufweist, die sich in einem Niveau zwischen der Oberfläche und den höchsten Punkten der Vorsprünge befindet.

Dieses bekannte Verankerungsteil weist also ein Design der Prothese (Struktur I.Ordnung) bis zu einer Mikrostruktur (Struktur III.Ordnung) auf, die durch die Vorsprünge auf der Oberfläche definiert ist. Die Verankerungsteile aus der DE-A-27 30 004 sollten infolge dieser Vorsprünge eine bessere Vernetzung des Knochengewebes in den Zwischenräumen zwischen den Vorsprüngen und somit eine widerstandsfähige Verankerung des Knochengewebes, vorzugsweise ohne Bindemittel, bewirken.

Die Oberflächengestaltung dieses bekannten Verankerungsteils weist jedoch den Nachteil auf, daß sie morphologisch nicht optimal strukturiert ist und keine formgebenden Elemente für die knochenbildende Zelle und für die tragende Knochenschale vorgegeben sind. Dazu kommt, daß die Oberflächenstrukturen nicht, auch nicht zum Teil, resorbierbar sind. Die Haftung der Knochenzellen am Basiskörper ist deshalb nicht so gut wie bei resorbierbaren Oberflächen. Weiterhin ist die bioaktive und die chemotaktische Wirkung (Knocheninduktion) bei nicht resorbierbaren Oberflächenbeschichtungen nicht in dem Maße gegeben, wie bei resorbierbaren Oberflächenbeschichtungen, zumal, wenn ihnen noch Wirkstoffe zugesetzt sind. Schließlich kann die Mikrostruktur der Oberfläche des aus der DE-A-27 30 004 bekannten Verankerungsteils keine Zusätze, wie Hämostyptika, knochenbildende Substanzen, Antibiotika, gefäßwirksame Substanzen und knochenwirksame Hormone aufnehmen.

Der größte Nachteil ist jedoch darin zu sehen, daß die Oberflächenstruktur die Ausbildung durchgehender und tragender Gewölbekonstruktionen des Knochens nicht ermöglicht.

In der DE-A-26 20 907 ist eine Prothesenschaftbeschichtung aus resorbierbaren keramischen Werkstoffen auf der Basis von Calciumphosphaten und nicht resorbierbaren Kunststoffen beschrieben. Bei der Resorption des keramischen Werkstoffs soll ein durchgängig poröses Gefüge aus Kunststoff entstehen, auf dessen inneren Porenoberflächen bioaktivierende Reste der Keramik zurückbleiben.

Eine nicht resorbierbare Kunststoffmatrix hat jedoch den Nachteil, daß der Kunststoff durch die im Interface auftretenden Scherkräfte zerrieben wird und das Abriebmaterial nicht resorbiert werden kann, was zu Entzündungen führt und die Lockerung der Prothese zur Folge haben kann. Ein weiterer wesentlicher Nachteil der Beschichtung gemäß DE-A-26 20 907 liegt darin, daß sich die als resorbierbar bezeichneten Keramikpartikel mit dem nicht resorbierbaren Kunststoff vollsaugen, was zu einer weiteren Verringerung der Resorbierbarkeit der gesamten Beschichtung führt. Deshalb kann der die Prothesenverankerung umgebende Knochen nicht ausreichend tief und schnell an den Basiskörper (Stütze) heranwachsen, was einen Festigkeitsverlust zur Folge hat.

Aus der US-A-3 855 638 ist eine Oberflächenstrukturierung des Verankerungsteiles einer Schaftprothese bekannt.
Auf einem Metallsubstrat ist eine 100 - 1000 $\mu$m dicke poröse Beschichtung aus demselben Metall aufgebracht, die aus im wesentlichen kugelförmigen Metallteilchen mit einer Größe von etwa 50 bis 150 $\mu$m besteht, zwischen denen Poren mit einer Größe von 20 bis 200 $\mu$m verteilt sind. Die Größenordnung und Verteilung der kugelförmigen Formelemente gemäß der US-A-3 855 638 wie auch die der US-A-4 206 516 ist jedoch wenig wirkungsvoll.

Das formgebende Element für die knochenbildende Zelle liegt unter 50 $\mu$m, vorzugsweise bei einer Größe von 15 - 30 $\mu$m, und das formgebende Element der tragenden Knochenbälkchen liegt bei 500 - 1000 $\mu$m. Mit der Größenverteilung der nicht resorbierbaren Formelemente der Beschichtung gemäß der US-A-3 855 638 und der US-A-4 206 516 ist es nicht möglich, schnelle und langfristig haltende knöcherne Verankerungen zu erzeugen. Die Zwischenräume ermöglichen zwar, wie dort aufgeführt, das Einwachsen von Faserstrukturen, auch von Geflechtknochen, nicht jedoch das Einwachsen und vor allem Durchwachsen von tragenden reifen Knochenstrukturen, für die größere Zwischenräume benötigt werden.

Aus der DE-A-26 20 890 ist eine partiell oder vollständig resorbierbare Knochenersatz-, Knochenverbund- oder Prothesenverankerungsmasse bekannt. Die Masse besteht, soweit sie resorbierbar ist, aus einer Mischung von Kalziumphosphaten mit weichen, plastischen, organischen Materialien. Die Masse soll als knetbare plastische Masse implantierbar sein, wobei eine Aushärtung der Masse nach der Implantation nur bei den lediglich partiell resorbierbaren Massen vorgesehen ist. Mit der Masse gemäß DE-A-26 29 890 soll Knochengewebe ersetzt werden können, und es soll der teilweise Ersatz bzw. das periphere oder totale Durchwachsen des aus der Masse gebildeten Implantates durch körpereigenes Gewebe erreicht werden.

Der Erfindung liegt die Aufgabe zugrunde, einen Knochenersatzwerkstoff zu schaffen, der sich zur Beschichtung bzw. Oberflächenstrukturierung von Knochenimplantaten sowie auch als geformtes Vollimplantat eignet, und der von tragenden Knochenstrukturen rasch und tief durchwachsen wird und deshalb stabile und beanspruchbare Implantate ergibt.

Diese Aufgabe wird aufgrund des überraschenden Befundes gelöst, daß Schichtungen von Elementarkörpern, welche in netzartigem Verbund stehen und in ihrem Hohlraumsystem nahezu morphologisch ideale Zwischenräume belassen, die gegebenenfalls mit einer Beschichtungsmasse ausgefüllt sind, die ihrerseits aus resorbierbaren Füllkörpern auf der Basis fester Calciumverbindungen und einem resorbierbaren Bindemittel besteht, einen Knocheneinwuchs induzieren und zur Ausbildung eines tragenden Knochengewölbes führen.

Gegenstand der Erfindung ist somit ein Knochenersatzwerkstoff mit den Merkmalen der Patentansprüche sowie Vollprothesen, Verankerungsteile für Gelenkprothesen, Überzüge für Knochen-oder Gelenkimplantate, Knochendübel und Markhöhlenverschlüsse aus derartigem Knochenersatzwerkstoff.

Der Knochenersatzwerkstoff der Erfindung weist sowohl eine charakteristische Struktur III.,als auch eine solche IV.Ordnung auf. Deshalb wird nicht nur in den Grenzflächen ein Knocheneinwuchs bewirkt, sondern auch die Ausbildung einer tragenden knöchernen Struktur ermöglicht, da die einwachsenden Knochenbälkchen zu Bogengewölben zusammenwachsen können.

Die Erfindung wird nachstehend mit Bezug auf die Zeichnung näher erläutert. Es zeigen:

Fig. 1         zwei Kugeln der Struktur IV. Ordnung, die die Basis eines Osteoblasten bilden,

Fig. 2         schematisch eine dichte Kugelpackung III. Ordnung mit einer kugelförmigen Oberflächenstrukturierung IV. Ordnung und angedeutetem Knocheneinbau,

Fig. 3         schematisch zwei kugelförmige Elementarkörper mit einer Beschichtungsmasse mit kugelförmigen Füllkörpern und faserverstärkter Matrix,

Fig. 4         eine Ansicht entsprechend Fig. 3 mit strumpfförmigem, durch die Matrix imprägniertem Füllkörper,

Fig. 5         eine Ansicht entsprechend Fig.

4 mit kugelkettenförmigem Füllkörper,

Fig. 6 und 7 einen Knochendübel und einen Markhöhlenverschluß aus dem erfindungsgemäßen Knochenersatzwerkstoff, und

Fig. 8 bis 10 weitere Ausführungs- und Verwendungsbeispiele des Werkstoffes. Zunächst wird die Ausbildung der Struktur III.Ordnung im erfindungsgemäßen Knochenersatzwerkstoff erläutert.

Überraschenderweise wurde gefunden, daß Probenkörper, die ausschließlich aus Schichtungen von Elementarkörpern bestimmter Gestalt und Größe bestehen, vollständig vom Knochen durchwachsen werden und daß diese Knochenstruktur im Schnittbild kaum von der physiologischen Spongiosaarchitektur unterschieden werden kann.

Es hat sich gezeigt, daß insbesondere geschichtete Kugelnetze einer Größe zwischen 200 und 3000 µm interkorpuskuläre Zwischenräume zwischen sich ergeben, deren Auffüllung mit Knochen nicht nur sehr viel schneller erfolgt als bei jeder anderen Beschichtung, sondern darüberhinaus auch eine nahezu physiologische und tragfähige Struktur III.Ordnung (Bälkchenarchitektur) ergibt. Die auf diese Weise erreichte Oberflächenvergrößerung führt zu einer riesigen tragenden und kraftaufnehmenden Oberfläche, welche in idealer Weise zur Resultierenden der einwirkenden Kraft nahezu trajektoriell ausgerichtet ist.

Knochenersatzwerkstoff, welcher vollständig aus geschichteten korpuskulären Netzwerken aufgebaut und in seiner Gesamtelastizität an den Knochen angepaßt ist, wird in sehr kurzer Zeit (12 Wochen) vollständig von Knochen durchwachsen und ergibt einen dauerhaften knöchernen Verbund.

Es hat sich gezeigt, daß eine Biegebeanspruchung eines solchen Implantates durch Verteilung über die gesamte Oberfläche abgefangen wird und daß Ermüdungsfrakturen aufgrund der riesigen Oberfläche und der dadurch geringen mechanischen Beanspruchung der Grenzfläche nicht mehr auftreten.

Darüberhinaus hat der Knochenersatzwerkstoff der Erfindung den großen Vorteil, daß die Masse des Gesamtimplantates erheblich verringert ist und die primäre Kopflastigkeit, die alle im Moment im Handel befindlichen Prothesen auszeichnet und die mit der Zeit zur Auslockerung dieser Prothesen führt, vermeidet.

Aufgrund der vorzugsweise kugelförmigen Struktur der Elementarkörper werden Kantenphänomene und Streßkonzentrationen vermieden, und es kommt zu einer gleichmäßigen Kraftverteilung über die Knochenoberfläche, was darin zum Ausdruck kommt, daß sich reife lamelläre Knochenstrukturen mit einer strengen parallelfaserigen und geschichteten Anordnung ausbilden.

Im Gegensatz zu einer Vollschaftprothese, welche durch einzelne Kugeln an ihrer Oberfläche strukturiert ist, weisen die geschichteten, netzförmigen Kugelverbände des Knochenersatzstoffes der Erfindung eine mechanisch stimulierende Wirkung auf die knochenbildende Zelle aus, was ebenfalls darin zum Ausdruck kommt, daß selbst die in die Tiefe eingewachsenen Knochenstrukturen noch reife lamelläre, d.h. biomechanisch beanspruchbare Knochenstruktur aufweisen.

Zu einem beschleunigten Knocheneinwuchs kommt es insbesondere dann, wenn größere Kugeln mit kleineren Kugeln kombiniert werden. Ein Durchmesserverhältnis von 2:1 bis 3:1 ist besonders günstig. Beispielsweise können Kugeln mit einem Durchmesser von 200 bis 1000 µm, im Mittel 500 µm, mit Kugeln mit einem Durchmesser von 800 bis 3000 µm, im Mittel 1000 µm, vermischt sein. Vorzugsweise weisen die kleineren Kugeln einen Durchmesser von 300 bis 700 µm, besonders bevorzugt 450 - 550 µm, und die größeren Kugeln einen Durchmesser von 800 bis 2000 µm, besonders bevorzugt 900 - 1200 µm auf. Dabei weisen die kleineren Kugeln einerseits und die größeren Kugeln andererseits vorzugsweise alle etwa den gleichen Durchmesser, also z.B. 500 µm und 1000 µm auf. Um die kleineren Kugelelemente kommt es schneller zu einer Knochenanlagerung, während die tragenden Knochengewölbe sich um die großen Kugelelemente ausbilden. Dadurch, daß alle Kugeln untereinander in starrer Verbindung oder zumindest in einstellbarer, elastisch verformbarer Verbindung zueinander stehen, kann eine fast natürliche Knochenkonstruktion erreicht werden, die mit den dazwischen sich ausbildenden Gefäß-Markräumen eine dauerhafte Intergration des Verankerungsteiles gewährleistet.

Eine relativ starre Verbindung ist dann gegeben, wenn jede Kugel mit mindestens drei benachbarten Kugeln in festem Kontakt steht. Je nach Struktur des Stützgerüstes kann jede Kugel auch mit mehr als drei, beispielsweise vier, sechs oder acht benachbarten Kugeln in festem Kontakt stehen, wodurch sich ein starres Gerüst ergibt.

In der Struktur III.Ordnung werden im Knochenersatzwerkstoff der Erfindung die Elementarkörper, die vorzugsweise Kugeln sind, durch Fäden aus resorbierbaren organischen Polymeren mit einer Dicke von 50 bis 300 µm zusammengehalten, oder sie bilden dreidimensionale dichteste Packungen.

Die Elementarkörper bestehen aus einem resorbierbaren Stoff, beispielsweise einem Stoff, aus dem auch die Struktur IV. Ordnung aufgebaut ist, wie Apatit, Tricalciumphosphat, einem Polypeptid, einem Polyactat, Polyglykolat oder aus einem ihrer Cokondensate, Gelatine, Kollagen oder aus einer Calciumverbindung als Matrix und vorzugsweise hochporösen Tricalciumphosphat- oder Hydroxylapatitpartikeln oder Partikeln einer verwandten Calciumverbindung als Füllkörper.

Auch wenn die Elementarkörper nicht kugelförmig sind, werden vorzugsweise Elementarkörper mit zwei verschiedenen Größen gemischt, wobei die vorstehenden Durchmesser dann etwa jeweils als mittlere Durchmesser definiert sind.

Die Kugeloberflächen können mikrostrukturiert sein, wobei die Mikrostrukturierung z.B. in Form von Kugeln oder Kugelabschnitten, wie Halbkugeln mit einem Durchmesser von 15 bis 30 $\mu$m ausgebildet ist.

Im Knochenersatzwerkstoff der Erfindung können die Zwischenräume des Elementarkörperaufbaus der Struktur III.Ordnung und/oder seine Oberfläche mit einer Beschichtungsmasse angefüllt bzw. bedeckt sein, die die Besonderheit aufweist, daß sie vollständig resorbierbar ist (sowohl Matrix als auch Füllkörper).

Die Mikrostrukturierung der Kugeloberflächen (III.Ordnung) stellt (falls vorhanden) zusammen mit der charakteristischen Beschichtungsmasse die Struktur IV.Ordnung (Ultrastruktur) des Knochenersatzwerkstoffes der Erfindung dar.

Diese Beschichtungsmasse (Struktur IV.Ordnung) wird nachstehend im einzelnen erläutert.

In einer Ausführungsform bestehen die Füllkörper aus hochporösen Kugelpartikeln mit einem Durchmesser von 10 bis 200 $\mu$m, vorzugsweise 15 bis 50 $\mu$m, besonders bevorzugt 15 - 30 $\mu$m, optimal etwa 20 $\mu$m. Diese Kugelpartikel weisen ein Porenvolumen von 25 bis 65%, vorzugsweise über 40%, auf. Aufgrund der erfindungsgemäßen Ultrastruktur wird die einzelne knochenbildende Zelle (Osteoblast) bzw. der Zellverband (Osteoblastenlayer) zum Aufbau bestimmter Knochenbälkchen angeregt. Weiterhin wird aufgrund der Struktur des erfindungsgemäßen Knochenersatzwerkstoffs die einzelne Knochenzelle angeregt. Dadurch erfolgt ein sehr rascher Einwuchs des Knochens in den Ersatzwerkstoff.

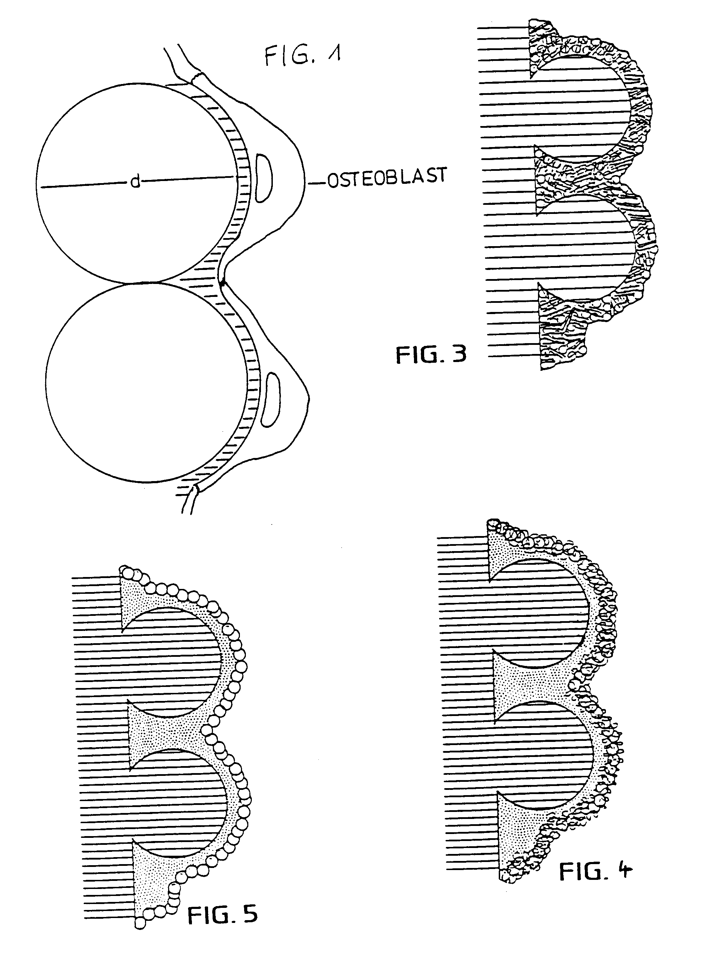

Es hat sich gezeigt, daß fest verankerte Kugeln oder Kugelteilflächen in der Größe von 15 bis 50 $\mu$m, insbesondere von 20 $\mu$m, die optimal strukturierte Oberfläche (Ultrastruktur IV.Ordnung) darstellen, die ein Osteoblast als seine Basis erkennen kann; vgl. Fig.1. Der Durchmesser d der Kugelteilchen beträgt vorzugsweise etwa 20 $\mu$m und ist in der Größenordnung eines Osteoblasten.

Die Poren der hochporösen Füllkörper können mit einem resorbierbaren und körperverträglichen Stoff gefüllt sein. Dieser Stoff ist vorzugsweise das verwendete Bindemittel. Spezielle Beispiele für verwendbare Bindemittel sind Polypeptide, Polylactate, Polyglykolate oder Cokondensate davon, Gelatine, Kollagen und Calciumverbindungen. Die Füllkörper sind vorzugsweise härter als das Bindemittel.

Weiterhin wurde erfindungsgemäß gefunden, daß eine organische Matrix zu einer sehr viel schnelleren Ausbildung einer Osteoblastenschicht führt als eine Metall- oder Keramikoberfläche. Es wurde auch gefunden, daß die Keramikoberfläche zwar besser kolonisiert wird als eine Metalloberfläche; die Oberfläche von gesintertem Apatit ist aber nochmals einer rascheren Zellkolonisation zugänglich als eine Keramikoberfläche. Es wurde auch gefunden, daß eine Kollagenmatrix noch rascher durch Zellen kolonisiert wird (besiedelt wird) als eine reine Keramik- oder eine reine Apatitoberfläche Die besten Kolonisationsraten werden erzielt, wenn die organischer Matrix (z.B.Kollagen) mit kleinen Kügelchen aus Apatit bestückt ist. Diese Beschichtungsmasse stellt eine im Sinne dieser Erfindung bevorzugte Ausführungsform der Struktur IV.Ordnung (Ultrastruktur) dar.

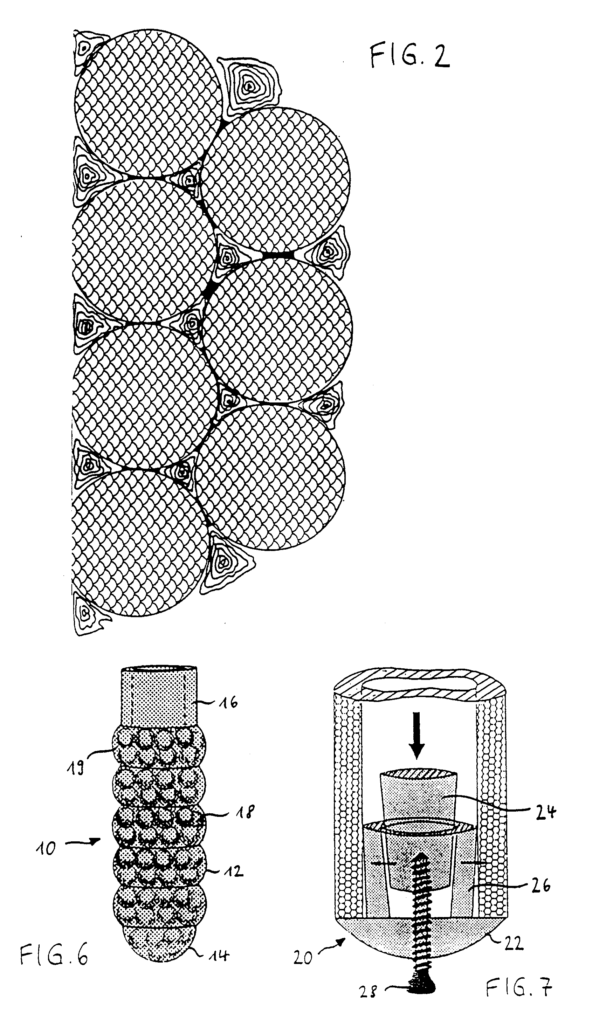

Die erfindungsgemäße Beschichtungsmasse ist vollständig resorbierbar. Eine vollständig resorbierbare Matrix hat gegenüber einer nicht resorbierbaren Matrix ganz wesentliche Vorteile. Sie wird in den Zonen der Beanspruchung sehr schnell resorbiert und durch Knochen ersetzt. Durch eine dichte Kugelpackung III.Ordnung (vgl. Fig. 2) kann in Verbindung mit einem dünnen Kollagenüberzug, der über den Kugeln verbleibt, in sehr kurzer Zeit eine geschlossene Osteoblastenschicht (Osteoblastenlayer) erhalten werden. Diese Osteoblastenschicht ist in der Lage, einen lamellären, stark beanspruchbaren Knochen zu bilden. Durch die Dichte der Kugelpackung und ihre konkrete Größe wird somit erreicht, daß der Anordnung von knochenbildenden Zellen primär eine bestimmte Struktur vorgegeben wird, so daß eine Umordnung der Zellen und der sich bildenden Knochenbälkchen nicht erforderlich wird. Dadurch wird die Zeit der Resorption und Knochenneubildung (Remodelling) eingespart.

In einer Ausführungsform des Knochenersatzwerkstoffes hat ein Teil der Füllkörper der Beschichtungsmasse Faserform. Vorzugsweise bestehen die faserförmigen Füllkörper aus verschieden langen Fasern, die eine Dicke von 100 bis 300, insbesondere etwa 200 $\mu$m aufweisen. Die Fasern sind vorzugsweise mehr als 2 bis 15 mm lang, besonders bevorzugt mindestens 3 mm und höchstens 10 mm lang, mit einer optimalen Länge von etwa 4 bis 5 mm.

Beispiele für Stoffe, aus denen die faserförmigen Füllstoffe bestehen können, sind Kohlenstoff, Kollagen, Polypeptide, Polyacetate, Polyglykolate oder deren Cokondensate, Gelatine oder Catgut. Der Anteil der faserförmigen Füllkörper kann etwa 5 bis 15%, vorzugsweise etwa 10% betragen.

In einer besonderen Ausführungsform der Erfindung sind die faserförmigen Füllkörper Bestandteil eines geschlossenen Netzes, das die Matrix armiert.

Ein Netzverband kann auch aus Kugelketten bestehen, in denen die Kugeln einen Durchmesser von 15 bis 50 $\mu$m, insbesondere etwa 20 $\mu$m aufweisen, wobei die Kugeln so dicht gelagert sind, daß sie in einem dreidimensionalen Verbund aneinanderstoßen. Die Netze können als Strumpf, insbesondere als mehrschichtiger Strumpf angeordnet sein.

Die Herstellung des eine Beschichtungsmasse aufweisenden Knochenersatzwerkstoffes der Erfindung wird nachstehend am Beispiel von Kollagen als vollständig resorbierbares Bindemittel erläutert. Nach einem üblichen Verfahren wird aus tierischen Knochen eine Kollagenmasse hergestellt, die wie ein Leim mit kugelförmigem Apatit oder TCP versetzt werden kann (der Ausdruck "Apatit" bedeutet in der Erfindung vorzugsweise "Hydroxylapatit", "TCP"bedeutet "Tricalciumphosphat"). Durch Anwendung von Infraschall, Schüttelrührwerk und/oder anderen Rührwerken kann eine dichte Kugelpakkung erzeugt werden. Mit dieser Beschichtungsmasse wird dann das Stützgerüst aus den Elementarkörpern imprägniert. Dabei wird ein Aufbau erhalten, in dem die erfindungsgemäßen Strkturen III. und IV.Ordnung kombiniert sind. Dieser Aufbau stellt einen bevorzugten Knochenersatzwerkstoff der Erfindung dar.

Ein Knochenersatzwerkstoff III.Ordnung, kombiniert mit der Ultrastruktur IV.Ordnung, kann jedoch auch auf folgende Weise erhalten werden: Aus der oben beschriebenen und mit Apatit bzw. TCP bestückten Kollagenmasse werden Kugeln der optimalen Größe zwischen 200 und 3000 $\mu$m hergestellt und auf einem resorbierbaren Faden zu Kugelketten aufgereiht. Diese Kugelketten werden in einer Rundstrickmaschine zu fortlaufenden Strümpfen verarbeitet, wobei ein Kugelkettennetzwerk entsteht. Von diesem Kugelkettennetzverband können durch Ineinanderstülpen verschiedene Formen von Implantaten, z.B. zur Behandlung von Knochendefekten, hergestellt werden.

Die mechanische Stabilität eines erfindungsgemäßen Knochenersatzwerkstoffes kann dadurch erheblich verbessert werden, daß Fäden oder Fasern verschiedener Länge oder Fadennetze bzw. Fadengewebe der Matrix einverleibt sind; vgl. Fig 3. Derartige Knochenersatzwerkstoffe stellen ebenfalls bevorzugte Ausführungsformen dar.

Besonders günstig ist die Ausführungsform, bei der ein gestrickter Strumpf oder ein Netz in einzelnen oder mehreren Schichten als Füllkörper verwendet und mit dem Bindemittel imprägniert wird. Die auf diese Weise erreichten Beschichtungen der Verankerungsteile der Prothesen sind ebenfalls bevorzugt. Sie erreichen eine sehr ausgeprägte mechanische Widerstandsfähigkeit; vgl. Fig 4.

Eine besonders günstige Wirkung für die Stimulation der Knochenbildung kann bei diesen Strümpfen auch dadurch erreicht werden, daß sie in Form von Kugelketten ausgebildet sind; vgl. Fig 5.

Solche Kugelketten können dadurch erhalten werden, daß man bei der Extrusion des Fadens kleine (15 bis 30 $\mu$m, vorzugsweise 20 $\mu$m) Kügelchen aus Apatit in den Faden einschließt. Derartige Ausführungsformen sind ebenfalls besonders bevorzugt. Anstelle der einschichtigen Kugelkette gemäß Fig. 5 können auch mehrschichtige, dreidimensionale Kugelkettenverbunde verwendet werden. Figur 6 zeigt ein Beispiel eines Knochendübels 10 zur Verankerung von Knochenschrauben aus dem erfindungsgemäßen Knochenersatzwerkstoff. Der Knochendübel 10 weist fünf Ringe oder Wülste 12, ein etwa halbkugelförmiges vorderes Ende 14 und einen zylindrischen Endabschnitt 16 auf. Kugelförmige Elementarkörper 18 bilden einen Matrix-Kugelverbund, der von einem Netz 19 armiert wird. Der Dübel 10 bewirkt nach dem Einbringen in den Knochen dessen Armierung und/oder Verstärkung. Er findet im umgebenden Knochen einen festen Halt und kann seinerseits eine eingedrehte Schraube langzeitig zugfest aufnehmen. Die Verankerung im Knochen erfolgt durch Aufquellen des Dübels oder Spreizen durch Eindrehen der Schraube. Der Dübel kann zum leichteren Einbringen in den Knochen mehrere Längs- oder Querschlitze aufweisen oder aus einem doppelwandigen Strumpfkäfig in der Form des Dübels gemäß Fig. 6 bestehen, in dem die Kugelketten gefangen und durch Verdrehen des Strumpfkäfigs zu einem Stützgerüst miteinander verbunden sind.

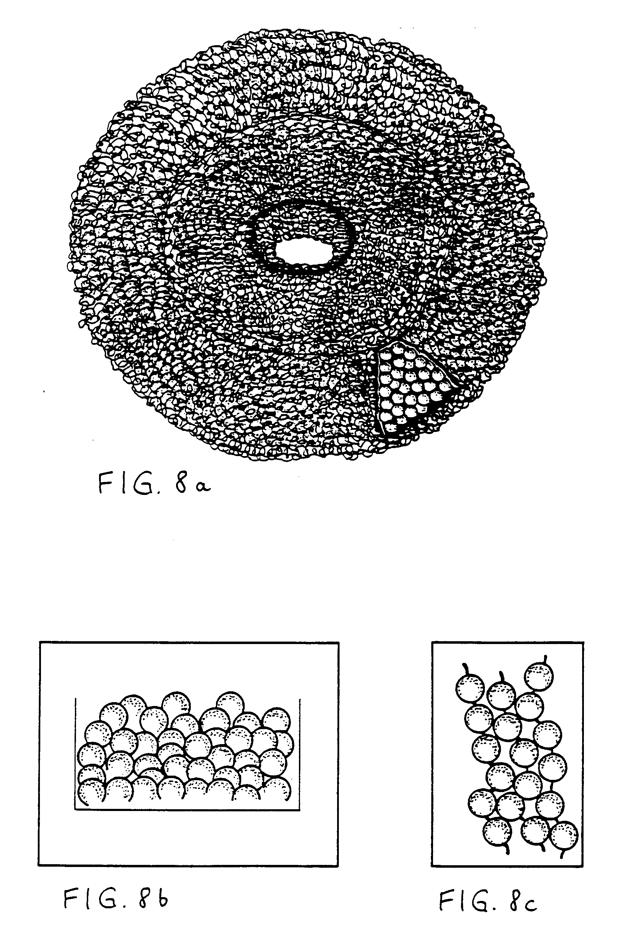

Figur 7 zeigt einen Markhöhlenverschluß 20 aus dem erfindungsgemäßen Knochenersatzwerkstoff. Der Verschluß 20 weist eine Kappe 22 über dem Markhöhlenstumpf, einen konusförmigen Pfropfen 24 und eine konusförmige Hülse 26 auf, in die der Pfropfen 24 durch Drehen einer Schraube 28 hineingezogen wird. Dadurch wird die Hülse 26 nach außen aufgespreizt und kommt in festen Eingriff mit dem Innern der in Fig. 7 schematisch mit Bienenwabenstrukur dargestellten Knochenröhre. Zur Behandlung von Knochendefekten und zum Auffüllen von Löchern im Knochen sind Verschlüsse aus dem erfindungsgemäßen Knochenersatzwerkstoff in Form einfacher, im Knochen aufquellender Zylinder, in Form massiver Lamellenzylinder oder in Form von Schrauben geeignet, die nach deren Entfernung in den Kanal einer Knochenschraube eingebracht werden. Derartige Verschlüsse bewirken eine hermetische Abdichtung gegenüber dem Innendruck der Knochenkanäle und/oder der Markhöhle, verhindern eine Nachblutung und Hämatonbildung und beschleunigen die Knochenheilung.

In Fig. 8a ist ein durch Ineinanderstülpen eines Kugelkettennetzverbandes hergestellter "Schwamm" zum Füllen von Knochendefekten dargestellt. Solche Formen sind von den "Scheuerschwämmchen" her bekannt. Durch Aufspreizen des Nabels dieser "Schwämmchen" können sie sehr leicht in Knochendefekthöhlen verblockt werden. Das Aufspreizen kann mittels eines konusförmigen oder dübelartigen Pfropfens durchgeführt werden. Fig. 8b und 8c zeigen schematisch eine perspektivische Aufsicht bzw. einen Querschnitt jeweils einer Schicht des dreidimensionalen Kugelkettennetzwerks.

Der Knochenersatzwerkstoff kann beispielsweise auch auf einen Prothesenstift oder -schaft aufgebracht werden, wobei der Überzug während des Trocknens auf dem Verankerungsteil der Prothese aufschrumpft und zu einem geschlossenen Kontakt zwischen Stützgerüst, Matrixmantel, Füllkörpern und dem Basismaterial führt. Eine derart beschichtete Prothese kann nun ohne bindenden Knochenzement in den Knochen eingesetzt werden. Es bilden sich bei entsprechender Abstützung in kurzer Zeit geschlossene knochenbildende Lagen auf der Oberfläche aus, die die Prothesenkomponente stabil knöchern abstützen können. Dabei bilden sich ausgesprochene Stütztrabekel aus, neben denen der Matrixüberzug resorbiert wird, so daß in der Folge der Knochen tief in die Oberflächenstruktur des Basiskörpers (Stützgerüst) einwachsen kann.

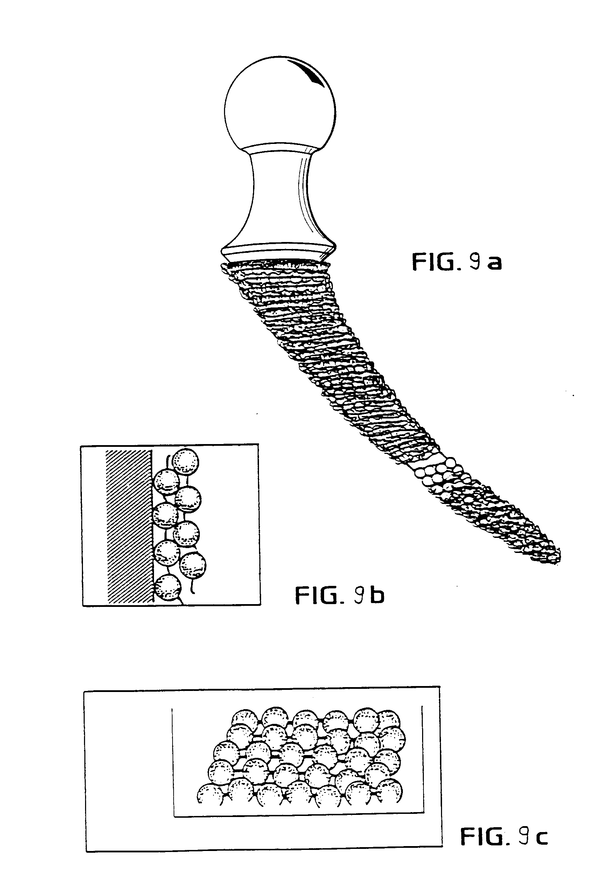

Fig. 9a zeigt ein erfindungsgemäßes Verankerungsteil einer Prothese mit darauf aufgebrachtem erfindungsgemäßen Werkstoff, bestehend aus einem Kugelkettenverbund. Fig. 9b zeigt einen schematischen Querschnitt mit dem Basismaterial und zwei Schichten des dreidimensionalen Verbundes, Fig. 9c eine Aufsicht auf die oberste Schicht des Verbundes.

Die Struktur III.Ordnung kann dabei sowohl Teil des Prothesenschaftes sein oder Teil des Überzuges. Besteht der resorbierbare Oberzug aus Formelementen III. und IV.Ordnung, so muß der metallene Prothesenschaft in der Weise strukturiert sein, daß er Tragrippen oder durchgehende Poren aufweist, um einen Knocheneinwuchs und eine knöcherne Unterfangung zu ermöglichen.

Durch die induzierte Wirkung der kleinen, beispielsweise von Kollagen bedeckten Kugeln aus Apatit kommt es zu einem sehr viel schnelleren Knochenanbau und -einbau des Implantates, als dies bei herkömmlichen Beschichtungen oder nicht beschichteten Implantaten der Fall ist.

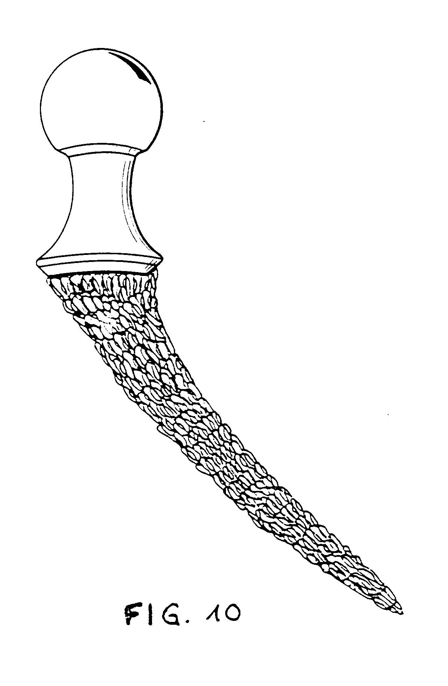

In einer besonderen Ausführungsform der Erfindung stellt der Knochenersatzwerkstoff der Erfindung selbst das vollständige Implantat oder auch Verankerungsteil einer Prothese dar. Dazu wird dem Werkstoff bei der Herstellung die entsprechende Form gegeben. Die Elementarkörper des Stützgerüstes können als dreidimensionales Netzwerk ausgebildet werden, das durch innere oder äußere Verstrebungen fest verspannt ist. Die einfachste Form stellt eine Kugelsinterung des Implantates dar. Eine solche Kugelpackung kann auch durch äußere Umschnürung erreicht werden (Kugelkäfig). Schließlich gelingt es auch, Kugelketten zu verarbeiten und entweder durch ein inneres, druckaufnehmendes Skelett, wie eine Tragrippenkonstruktion, oder einen "Pneu" zu verspannen, oder durch einzelne Schweißpunkte mit einstellbarer Elastizität zu versteifen. Durch die Zahl und Dichte der Schweißpunkte,durch die einzelne Kugeln starr miteinander verbunden werden können, läßt sich die Steifigkeit und Elastizität auch bestimmter Teile des Knochenersatzwerkstoffes gezielt einstellen. Fig. 10 zeigt ein Verankerurgsteil, bei dem das Netz sowohl als Armierung als auch - nach entsprechender Versteifung oder Verspannung - als Verankerungsteil selbst ausgebildet sein kann. Aus dem erfindungsgemäßen Knochenersatzwerkstoff können vorteilhaft auch Kunstknochen hergestellt werden, z.B. für Schulungszwecke und Operationskurse.

Ein dreidimensionales Netzwerk kann auch so verdreht oder verflochten werden, daß Implantate mit unterschiedlichen Elastizitäten, beispielsweise auch in verschiedenen Abschnitten des Implantats, resultieren. Der Knochenersatzwerkstoff der vorliegenden Erfindung kann in Form eines Überzuges, eines Köchers, Zylinders, einer Spiralfeder, einer Kugel oder in Form eines Schwammes gebracht werden.

Die Induktion der Knochenbildung kann durch Hinzufügen chemischer Wirkstoffe in die Matrixsubstanz noch erhöht werden. Beispielsweise sind chemotaktisch wirksame Stoffe aus Knochengrundsubstanz und nekrotischen Knochen bekannt, die sogenannte "bone morphogenic" Proteine enthalten, welche eine besonders induzierende Wirkung auf die Knochenbildung ausüben. Die Zugabe solcher Zusätze ist bevorzugt.

Es hat sich gezeigt, daß die Induktion der Knochenbildung dann besonders günstig ist, wenn die als Füllerpartikel eingesetzten hochporösen Kugelpartikel härter sind als die umgebende Matrix, so daß sie eine mechanisch stimulierende Wirkung auf die knochenbildende Zelle ausüben. Eine solche Ausführungsform ist ebenfalls bevorzugt.

Ein weiterer Vorteil des erfindungsgemäßen Knochenersatzwerkstoffs liegt darin, daß er eine beträchtliche Blndemittelmasse enthalten kann. Es hat sich gezeigt, daß diese eine recht beachtliche Kapazität mit guten Freisetzungsraten im Hinblick auf Wirkstoffzusätze aufweist. Es ist bekannt, daß Implantate, die der Körperabwehr aufgrund der fehlenden Vaskularisation nicht zugänglich sind, dadurch gefährdet sind, daß sie sehr leicht von Bakterien besiedelt werden. Dies kann dadurch verhindert werden, daß man dem Knochenersatzwerkstoff ein Antibiotikum zusetzt. Solche Antibiotikabeimengungen sind von den Knochenzementen her sehr gut erforscht. Die zementfreien Prothesen verfügten bislang nicht über diesen Schutz. Mit dem Knochenersatzwerkstoff aus resorbierbaren Stoffen ist es jedoch möglich, auch die zementfreien Prothesen und Implantate, gleich welcher Art, mit einem wirksamen Infektionsschutz prophylaktisch zu versorgen.

Die lokale Applikation anderer Medikamente, wie Hämostyptika, ist bei äußeren Behandlungen bekannt. Durch den Zusatz solcher Stoffe zum Knochenersatzwerkstoff der Erfindung können solche Wirkstoffe auch dort wirksam werden, wo die Organe einer äußeren Behandlung nicht zugängig sind. Hämostyptika als Bestandteile des Bindemittels sorgen für eine sofortige Blutstillung im knöchernen Lager. Gefäßwirksame Substanzen, wie Noradrenalin oder eines seiner Abkömmlinge, führen über die Gefäßverengung ebenfalls zu einem blutstillenden Effekt. Dadurch wird die Durchtränkung der Grenzschicht mit Blut verhindert, wodurch die mechanische Festigkeit der Grenzzone länger erhalten bleibt. Ferner können, wie bereits erwähnt, knocheninduzierende, chemotaktisch wirksame Substanzen dem Bindemittel beigemengt werden. Diese führen zu einem schnelleren Beginn der Knochenneubildung. Mit Substanzen vom Typ des Calcitonin sind Hormonapplikationen lokal wirksam, die verhindern, daß der neugebildete Knochen wieder abgebaut wird.

Die Beispiele erläutern die Erfindung.

Beispiel 1

Die Herstellung eines Knochenersatzwerkstoffes, bestehend aus einem dreidimensionalen Stützgerüst aus miteinander in Verbindung stehenden und definierte Zwischenräume umschliessenden Elementarkörpern, mit und ohne Beschichtungsmasse, kann auf folgende Weise hergestellt werden: In einem Extruder wird die resorbierbare organische Matrixsubstanz Polypeptid, z.B. Polyglycolat oder Polylactat, unter Zugabe verschiedener Zusatzstoffe als Weichmacher bis zur Schmelze erhitzt. Anschliessend werden die Füllerpartikel in Form hochporöser Tricalciumphosphatkugeln hinzugefügt und die gesamte Masse unter Erwärmung kräftig gerührt. Die Tricalciumphosphatkugeln werden vorzugsweise aus einem hochporösen gesinterten Tricalciumphosphat, welches im wesentlichen betawhitlocked Struktur aufweist, durch Mahlen in einer Kugelmühle erhalten.

Auf diese Weise entsteht durch Rühren eine Schmelze mit homogener Verteilung des Füllers. Durch Anlegen eines Druckes wird das Material der Schmelze durch feine Düsen ausgepresst und in Fällungsbädern als Faden mit einer Dicke von z.B. etwa 200 $\mu$m aufgerollt. Die Kugel- oder kugelähnlichen Strukturen auf den Fäden erhält man auf verschienden Weise. Einmal können die Kugeln größeren Durchmessers von z.B.etwa 1000 bis 3000 $\mu$m rein mechanisch aufgefädelt werden, zum anderen können sie, vor allem, wenn es sich um Kugeln kleineren Durchmessers von z.B. etwa 500 $\mu$m handelt, vorzugsweise dadurch erhalten werden, daß man den Durchmesser der stufenlos regulierbaren Düsenöffnung pulsierend verändert.

Auf diese Weise können durch Ausfällen der Schmelze im Bad fortlaufende Kugelketten mit einstellbarer Kugelgröße erreicht werden. An diesen Kugelketten können die gegebenenfalls mit Matrixsubstanz umgebenen kleineren Füllerpartikel mit einem Durchmesser von z.B. etwa 15 - 30 $\mu$m außen anhaften.

Die auf diese Weise hergestellten Kugelketten können dann in Endlos-Rundstrickmaschinen, beispielsweise Firma Müller in Weissenburg oder Firma Dubied in Neuenburg/Schweiz,zu endlosen Strümpfen verstrickt werden. Durch mehrfaches Ineinanderstülpen des Strumpfes erhält man ein sehr dichtes, dreidimensionales Geflecht von Kugelketten, welches äusserlich z.B. die Form eines Reifens hat, der in idealer Weise durch einen zentralen Spreizer beispielsweise in der Knochenhöhle verblockt werden kann.

Beispiel 2

Geschichtete korpuskuläre Netzwerke können auch durch Übereinanderstülpen von solchen Netzen erreicht werden, indem metallene Kugelketten zu Netzwerken verstrickt oder verschweisst, in wenigen oder mehreren Schichten übereinandergestülpt und durch Schweissverfahren versteift werden, so dass sich steuerbar verschiedene Elastizitäten des Gesamtimplantates einstellen lassen. In sehr einfacher Weise kann man solche Netze über einen Kern stülpen, so dass sie in Form eines Strumpfes diesen Kern voll umfassen. Nach Drehen des Strumpfes um z.B. 180° kann eine zweite Schicht darübergestülpt werden und so kann dieses Verfahren wiederholt werden, so dass insgesamt eine Übereinanderlagerung von mehreren Schichten dieses endlosen Kugelkettenstrumpfes erreicht wird.

In einem zweiten Verfahren wird dann dieses so erhaltene Gerüstwerk aus korpuskulären geschichteten Netzen verschweisst und nach einem genau vorher berechneten Plan versteift. Eine zusätzliche Versteifung kann entstehen dann durch Ausgiessen des Hohlraumes mit einer organischen Matrixsubstanz,mit oder ohne Füllerpartikel, welche die Struktur IV darstellen, entstehen. Matrix und Füllerpartikel sind dadurch ausgezeichnet, dass sie resorbierbar sind und mit Werkstoffen beschickt werden können.

Beispiel 3

Einzelne oder geschichtete Netzwerke auf Prothesenvollschäften oder gänzlich aus geschichteten korpuskulären Netzen aufgebaute Verankerungsteile lassen sich auf verschiedene Weise herstellen. Verankerungsteile bis zu zwei Schichten korpuskulärer Netze können nach herkömmlichen Verfahren kostengünstig hergestellt werden. Die Herstellung dieser Struktur III beruht auf dem Verfahren eines Gusses in einer verlorenen Form. Diese Form wird wiederum mit Hilfe von geschichteten Wachskugelnetzen als ausschmelzbares Modell hergestellt. Diese Verfahren sind aus der Dentaltechnik her bekannt und liegen allen einfachen Oberflächenstrukturierungen von gegossenen Metallschäften zugrunde. Das auf diese Weise erhaltene Stützgerüst wird zur Ausfüllung der Hohlräume noch mit einer organischen Matrix beschichtet. Das Beschichtungsmaterial kann vorzugsweise mit Füllerpartikeln beschickt werden und weist alle Merkmale der oben beschriebenen Struktur IV auf. Das Beschichten kann beispielsweise durch Eintauchen der Struktur III in eine Schmelze der Struktur IV erfolgen.

Beispiel 4

Der aus dem dreidimensionalen Stützgerüst der Struktur III aus Elementarkörpern bestehende Knochenersatzwerkstoff ohne Beschichtungsmasse ist auch als Ausgangsmaterial für Kunstknochen verwendbar. Die Elementarkörper werden zunächst in eine Ausgußmasse, beispielsweise aus Kunststoff, eingegossen und fest umschlossen. Danach werden die Elementarkörper aus der Ausgußmasse herausgelöst, so daß lediglich die dem Knochenaufbau ähnliche Struktur der Ausgußmasse verbleibt. In diesem Fall bestehen die Elementarkörper aus einem Material, das in einem Lösungsmittel chemisch lösbar ist, welches die Ausgußmasse nicht angreift, oder die Elementarkörper werden physikalisch, beispielsweise elektrolytisch, aus der Ausgußform entfernt.

**Patentansprüche**

1.  Knochenersatzwerkstoff aus einer resorbierbaren Substanz, dadurch gekennzeichnet, daß er ein dreidimensionales Stützgerüst aus miteinander in Verbindung stehenden und Zwischenräume umschließenden Elementarkörpern aufweist, welche aus der resorbierbaren Substanz bestehen und eine Größe von mindestens 200 $\mu$m aufweisen.

2.  Knochenersatzwerkstoff nach Anspruch 1, dadurch gekennzeichnet, daß das Stützgerüst aus mit den Elementarkörpern bestückten Fäden aus resorbierbaren organischen Polymeren mit einer Dicke von 50 - 300 $\mu$m und/oder aus einer dichtesten dreidimensionalen Packung der Elementarkörper aufgebaut ist.

3.  Knochenersatzwerkstoff nach Anspruch 1 oder 2, dadurch gekennzeichnet, daß die resorbierbaren Elementarkörper aus Tricalciumphosphat, Apatit, Kollagen, einem Polypeptid oder einem Gemisch zweier oder mehrerer dieser Werkstoffe bestehen.

4.  Knochenersatzwerkstoff nach Anspruch 1 bis 3, dadurch gekennzeichnet, daß die Elementarkörper Kugeln oder kugelähnliche Formkörper sind, deren Größe vorzugsweise zwischen 200 $\mu$m und 3000 $\mu$m liegt.

5.  Knochenersatzwerkstoff nach Anspruch 4, dadurch gekennzeichnet, daß die Kugeln unterschiedliche Durchmesser aufweisen, und bevorzugt Kugeln mit einem Durchmesser von 200 bis 1000 $\mu$m, im Mittel 500 $\mu$m, und solche mit einem Durchmesser von 800 bis 3000 $\mu$m, im Mittel 1000 $\mu$m, enthalten sind.

6.  Knochenersatzwerkstoff nach Anspruch 1 bis 5, dadurch gekennzeichnet, daß die Elementarkörper zum Teil oder alle starr miteinander in einer dreidimensionalen Verbindung stehen.

7.  Knochenersatzwerkstoff nach Anspruch 1 bis 6, dadurch gekennzeichnet, daß die einzelnen oder geschichteten Elementarkörper ein dreidimensionales Gerüst oder Netzwerk darstellen, das durch innere oder äußere Verstrebungen fest Verspannt ist, wobei das dreidimensionale Netzwerk vorzugsweise in sich verdreht oder verflochten oder versteift ist, und/oder nach dem Pneu-Prinzip durch Innendruck im Sinne einer zugfesten Hülle oder durch Streben im Sinne eines Skelettes von innen verspannbar ist.

8.  Knochenersatzwerkstoff nach Anspruch 1 bis 7, dadurch gekennzeichnet, daß die Oberflächen der Elementarkörper mikrostrukturiert sind, wobei die Mikrostrukturierung vorzugsweise in Form von Kugeln oder Kugelabschnitten mit Durchmessern von 15 - 30 $\mu$m ausge-

bildet ist.

9. Knochenersatzwerkstoff nach Anspruch 1 bis 8, gekennzeichnet, durch eine Beschichtungsmasse, die die Zwischenräume zwischen den Elementarkörpern des Stützgerüstes ausfüllt und/oder deren Oberfläche bedeckt, und die aus resorbierbaren Füllkörpern auf der Basis fester Calciumverbindungen und einem resorbierbaren Bindemittel (Matrix) besteht.

10. Knochenersatzwerkstoff nach Anspruch 1 bis 9, dadurch gekennzeichnet, daß die Füllkörper der Beschichtungsmasse hochporöse Kugelpartikel mit einem Durchmesser von 10 bis 200 $\mu$m, vorzugsweise zwischen 15 und 30 $\mu$m, und einem Porenvolumen von 25 - 65% sind, vorzugsweise hochporöse Tricalciumphosphat- oder Hydroxylapatitpartikel oder Partikel einer verwandten Calciumverbindung, deren Poren mit einem resorbierbaren und körperverträglichen Stoff gefüllt sind, wobei der resorbierbare, körperverträgliche Stoff das Bindemittel (Matrix) der Beschichtungsmasse ist, z.B. ein Polypeptid, ein Polylactat, Polyglykolat oder deren Cokondensate, Gelatine, Kollagen oder eine Calciumverbindung.

11. Knochenersatzwerkstoff nach Anspruch 10, dadurch gekennzeichnet, daß die Füllkörper härter als das Bindemittel sind.

12. Knochenersatzwerkstoff nach Anspruch 10 oder 11, dadurch gekennzeichnet, daß die Füllkörper einen Durchmesser von etwa 20 $\mu$m aufweisen, und vorzugsweise zum Teil Faserform haben, wobei die faserförmigen Füllkörper vorzugsweise aus verschieden langen Fasern zwischen 2 und 15 mm bestehen, die vorzugsweise eine Dicke von 100 - 300 $\mu$m, vorzugsweise 200 $\mu$m aufweisen.

13. Knochenersatzwerkstoff nach Anspruch 12, dadurch gekennzeichnet, daß die faserförmigen Füllkörper aus Kohlenstoff, Kollagen, Peptiden, Polylactat, Polypeptiden, Polyglykolat oder deren Cokondensaten, Gelatine oder Catgut bestehen, und vorzugsweise ein geschlossenes Netz bilden.

14. Knochenersatzwerkstoff nach Anspruch 1 bis 13, gekennzeichnet durch ein vorzugsweise mehrschichtiges Netz aus Ketten von Kugeln mit einem Durchmesser von 15 bis 50 $\mu$m, vorzugsweise etwa 20 $\mu$m wobei die Kugeln so dicht gelagert sind, daß sie in einem dreidimensionalen Verbund aneinanderstoßen.

15. Knochenersatzwerkstoff nach den Ansprüchen 1 bis 14, dadurch gekennzeichnet, daß die Matrix Zusätze aus der Gruppe Hämostyptika, knochenbildende Substanzen, zur Behandlung von Knocheninfektionen wirksame, Antibiotika, z.B. Gentamycin gefäßwirksame Substanzen, z.B. Noradrenalin und/oder eines seiner Abkömmlinge, und knochenwirksame Hormone, z.B. Calcitonin enthält.

16. Knochenersatzwerkstoff nach Anspruch 1 bis 15, dadurch gekennzeichnet, daß er in Form eines Köchers, Zylinders, einer Spiralfeder, einer Kugel oder eines Schwammes ausgebildet ist.

17. Verwendung des Knochenersatzwerkstoffes nach Anspruch 1 bis 16 zur Herstellung einer Vollprothese oder zur Herstellung eines Verankerungsteils einer Gelenkprothese oder zur Herstellung eines Überzugs auf einem Knochen-oder Gelenkimplantat zur Behandlung von Knochendefekten oder zum Gelenkersatz oder zur Herstellung eines Knochendübels zur Verankerung von Knochenschrauben oder zur Herstellung eines Markhöhlenverschlusses zum Abschluß der Markhöhle bei Amputationen.

**Claims**

1. Bone replacement material consisting of an absorbable substance, characterized in that the material comprises a three-dimensional supporting structure of elementary bodies in contact with one another and enclosing defined spaces, said elementary bodies consisting of the absorbable substance and being at least 200 $\mu$m in size.

2. Bone replacement material according to claim 1, characterized in that the supporting structure is made of threads, which contain the elementary bodies, of absorbable organic polymers with a thickness of 50 - 300 $\mu$m and/or of a three-dimensional, maximum density pack of the elementary bodies.

3. Bone replacement material according to claim 1 or 2, characterized in that the absorbable elementary bodies consist of tricalcium phosphate, apatite, collagen, a polypeptide or a mixture of two or more of these materials.

4. Bone replacement material according to any of claims 1 to 3, characterized in that the elementary bodies are spheres or sphere-like bodies, the size of which is preferably between 200

$\mu$m and 3000 $\mu$m.

5. Bone replacement material according to claim 4, characterized in that the spheres have different diameters and that the material preferably comprises spheres with a diameter of 200 to 1000 $\mu$m, with a mean of 500 $\mu$m, and spheres with a diameter of 800 to 3000 $\mu$m, with a mean of 1000 $\mu$m.

6. Bone replacement material according to any of claims 1 to 5, characterized in that some or all of the elementary bodies are in rigid, three-dimensional contact with one another.

7. Bone replacement material according to any of claims 1 to 6, characterized in that the individual or layered elementary bodies form a three-dimensional framework or network which is rigidly braced by internal or external struts, wherein the three-dimensional network is preferably twisted, interlaced or strengthened and/or can be braced like a tension-resistant enclosure by internal pressure according to the tire principle, or like a skeleton from within by girders.

8. Bone replacement material according to any of claims 1 to 7, characterized in that the surfaces of the elementary bodies are microstructured, wherein the microstructure preferably takes the form of spheres or spherical segments with diameters of 15 - 30 $\mu$m.

9. Bone replacement material according to any of claims 1 to 8, characterized by a coating mass which fills the spaces between the supporting structure's elementary bodies and/or covers their surface, and which consists of solid calcium compound-based absorbable fillers and an absorbable binding agent (matrix).

10. Bone replacement material according to any of claims 1 to 9, characterized in that the coating mass's fillers are highly porous spherical particles with a diameter of 10 to 200 $\mu$m, preferably between 15 and 30 $\mu$m, and a pore volume of 25 - 65%, preferably highly porous tricalcium phosphate or hydroxyl apatite particles or particles of a related calcium compound, the pores of which are filled with an absorbable and body compatible substance, wherein the absorbable, body compatible substance is the coating mass's binding agent (matrix), e.g. a polypeptide, a polylactate, a polyglycolate or their co-condensates, gelatine, collagen or a calcium compound.

11. Bone replacement material according to claim 10, characterized in that the fillers are harder than the binding agent.

12. Bone replacement material according to claim 10 or 11, characterized in that the fillers have a diameter of approximately 20 $\mu$m, and that some of the fillers preferably have a fibrous form, wherein the fibrous fillers preferably consist of fibers varying in length from 2 to 15 mm, which preferably have a thickness of 100 - 300 $\mu$m, preferably 200 $\mu$m.

13. Bone replacement material according to claim 12, characterized in that the fibrous fillers consist of carbon, collagen, peptides, polylactate, polypeptides, polyglycolate or their co-condensates, gelatine or catgut, and preferably form a closed network.

14. Bone replacement material according to any of claims 1 to 13, characterized by a preferably multi-layered network of chains of spheres with a diameter of 15 to 50 $\mu$m, preferably approximately 20 $\mu$m, wherein the spheres are so densely packed that they abut in a three-dimensional bond.

15. Bone replacement material according to any of claims 1 to 14, characterized in that the matrix contains admixtures of the groups consisting of hemostatic agents, bone-building substances, antibiotics effective in the treatment of bone infections, e.g. gentamycin, vasoactive substances, e.g. noradrenalin and/or one of its derivatives, and bone-active hormones, e.g. calcitonin.

16. Bone replacement material according to any of claims 1 to 15, characterized in that the material takes the form of a socket, a cylinder, a flat coil spring, a sphere or a sponge.

17. Use of the bone replacement material according to any of claims 1 to 16 for the manufacture of a full prosthesis, or for the manufacture of an anchoring part of a joint prosthesis, or for the manufacture of a coating on a bone or joint implant for the treatment of bone defects or as a joint replacement, or for the manufacture of a bone dowel for anchoring bone screws, or for the manufacture of a medullary cavity closure for closing off medullary cavities in the case of amputations.

**Revendications**

1. Matériau de remplacement d'os à base d'une substance résorbable, caractérisé en ce qu'il présente une charpente de support tridimensionnelle à base de corps élémentaires qui sont mutuellement en liaison, renferment des espaces intercalaires, sont constitués de la substance résorbable et présentent une dimension d'au moins 200 µm.

2. Matériau de remplacement d'os suivant la revendication 1, caractérisé en ce que la charpente de support est constituée de fils en polymères organiques résorbables, garnis des corps élémentaires et présentant une grosseur de 50 à 300 µm et/ou d'un entassement tridimensionnel très dense des corps élémentaires.

3. Matériau de remplacement d'os suivant l'une des revendications 1 et 2, caractérisé en ce que les corps élémentaires résorbables sont constitués de phosphate tricalcique, d'apatite, de collagène, d'un polypeptide ou d'un mélange de deux ou plusieurs de ces matériaux.

4. Matériau de remplacement d'os suivant l'une des revendications 1 à 3, caractérisé en ce que les corps élémentaires sont des sphères ou des corps analogues à des sphères dont la grandeur est de préférence comprise entre 200 µm et 3000 µm.

5. Matériau de remplacement d'os suivant la revendication 4, caractérisé en ce que les sphères présentent des diamètres différents et en ce qu'avantageusement des sphères ayant un diamètre de 200 à 1000 µm, en moyenne de 500 µm, et des sphères ayant un diamètre de 800 à 3000 µm, en moyenne de 1000 µm, sont contenues.

6. Matériau de remplacement d'os suivant l'une des revendications 1 à 5, caractérisé en ce que les corps élémentaires se trouvent en partie ou tous dans une liaison mutuelle tridimensionnelle rigide.

7. Matériau de remplacement d'os suivant l'une des revendications 1 à 6, caractérisé en ce que les corps élémentaires individuels ou en couches forment une charpente ou réseau tridimensionnel qui est croisillonné de manière solide par des entretoisements internes ou externes, le réseau tridimensionnel étant de préférence tordu ou entrelacé ou rigidifié sur soi et/ou pouvant être tendu de l'intérieur, selon le principe du pneu, par une pression interne dans le sens d'une enveloppe résistant à la traction ou par des entretoises dans le sens d'un squelette.

8. Matériau de remplacement d'os suivant l'une des revendications 1 à 7, caractérisé en ce que les surfaces des corps élémentaires sont microstructurés, la microstructuration étant réalisée de préférence sous la forme de sphères ou de segments de sphères ayant des diamètres de 15 à 30 µm.

9. Matériau de remplacement d'os suivant l'une des revendications 1 à 8, caractérisé par une masse d'enduction qui remplit les espaces intercalaires entre les corps élémentaires de la charpente de support et/ou qui recouvre sa surface et qui est constituée de corps de remplissage résorbables à base de composés de calcium solides et d'un liant résorbable (matrice).

10. Matériau de remplacement d'os suivant l'une des revendications 1 à 9, caractérisé en ce que les corps de remplissage de la masse d'enduction sont des particules sphériques hautement poreuses ayant un diamètre de 10 à 200 µm, de préférence compris entre 15 et 30 µm, et un volume poreux de 25 à 65 %, de préférence des particules de phosphate tricalcique ou d'hydroxyle apatite hautement poreuses ou des particules d'un composé de calcium apparenté, dont les pores sont remplis d'une substance résorbable et compatible avec le corps, la substance résorbable, compatible avec le corps, étant le liant (matrice) de la masse d'enduction, par exemple un polypeptide, un polylactate, un polyglycolate ou des produits de condensation de celui-ci, des gélatines, du collagène ou un composé de calcium.

11. Matériau de remplacement d'os suivant la revendication 10, caractérisé en ce que les corps de remplissage sont plus durs que le liant.

12. Matériau de remplacement d'os suivant l'une des revendications 10 et 11, caractérisé en ce que les corps de remplissage présentent un diamètre d'environ 20 µm et ont de préférence partiellement une forme de fibres, les corps de remplissage fibreux étant de préférence constitués de fibres différemment longues comprises entre 2 et 15 mm, qui présentent de préférence une grosseur de 100 à 300 µm, de préférence de 200 µm.

13. Matériau de remplacement d'os suivant la revendication 12, caractérisé en ce que les corps de remplissage fibreux sont constitués de carbone, de collagène, de peptides, de polylactate, de polypeptides, de polyglycolate ou de ses produits de cocondensation, de gélatines ou de catgut, et en ce que de préférence ils forment un réseau ferme.

14. Matériau de remplacement d'os suivant l'une des revendications 1 à 13, caractérisé par un réseau, de préférence en plusieurs couches, constitué de chaînes de sphères ayant un diamètre de 15 à 50 $\mu$m, de préférence d'environ 20 $\mu$m, les sphères étant supportées si densément qu'elles sont contiguës dans un composite tridimensionnel.

15. Matériau de remplacement d'os suivant l'une des revendications 1 à 14, caractérisé en ce que la matrice contient des additifs du groupe des hémostyptiques, des substances ostéogènes, des antibiotiques actifs pour le traitement des infections osseuses, par exemple de la gentamycine, des substances actives sur les vaisseaux, par exemple de la noradrénaline et/ou un de ses dérivés, et des hormones actives sur les os, par exemple de la calcitonine.

16. Matériau de remplacement d'os suivant l'une des revendications 1 à 15, caractérisé en ce qu'il est réalisé sous la forme d'un carquois, d'un cylindre, d'un ressort spiralé, d'une sphère ou d'une éponge.

17. Utilisation du matériau de remplacement d'os suivant l'une des revendications 1 à 16, pour la réalisation d'une prothèse complète ou pour la réalisation d'une pièce d'ancrage d'une prothèse d'articulation ou pour la réalisation d'un revêtement sur un implant d'os ou d'articulation en vue du traitement de défauts osseux ou pour le remplacement d'une articulation ou pour la réalisation d'une cheville osseuse destinée à l'ancrage de vis ou pour la réalisation d'une fermeture de cavité médullaire destinée à fermer la cavité médullaire dans le cas d'amputations.

FIG. 1

OSTEOBLAST

FIG. 3

FIG. 5

FIG. 4

FIG. 2

FIG. 6

16

19

10

18

12

14

FIG. 7

24

26

22

20

28

FIG. 8a

FIG. 8b

FIG. 8c

FIG. 9 a

FIG. 9 b

FIG. 9 c

FIG. 10